# EUROPEAN PATENT APPLICATION

(11) **EP 1 801 228 A1**
(43) Date of publication of application: **27.06.2007**
(21) Application number: 05767289.1
(22) Date of filing: 01.08.2005
(51) Int. Cl.: C12P 41/00

(54) **PROCESS FOR PRODUCTION OF ENANTIOMER-ENRICHED COMPOUNDS**

(30) Priority: 06.08.2004 JP 2004230139
(71) Applicant: Kaneka Corporation, Kita-ku Osaka-shi, Osaka 530-8288 (JP)
(72) Inventor: IWASAKI, Akira, 6750068 (JP); WASHIDA, Motohisa, Hyogo 655-0893 (JP); HASEGAWA, Junzou, Hyogo 6740057 (JP)
(74) Representative: HOFFMANN EITLE
(86) International application number: PCT/JP2005/014007
(87) International publication number: WO 2006/013802

(57) **Abstract**

The present invention relates to a production method of an enantiomer-enriched compound, which includes the following (1) and (2):
(1) contacting an enantiomer mixture with a stereoselective dehydrogenase, for which NAD+ (nicotinamide adenine dinucleotide) is a coenzyme, thereby preferentially oxidizing one enantiomer and leaving the other enantiomer to form NADH, and
(2) contacting the NADH formed by the oxidation reaction in (1) with a water-forming NADH oxidase stable under oxidative conditions to convert the NADH to NAD+.

According to the present invention, in the production of an enantiomer-enriched compound from an enantiomer mixture, which uses a dehydrogenase, NAD+ can be regenerated even without addition of a stabilizer and an enantiomer-enriched compound can be obtained efficiently.

SEQUENCE LISTING

<110>  Kaneka Corporation

<120>  PROCESS FOR PRODUCTION OF ENANTIOMER-ENRICHED COMPOUNDS

<130>  B040392W001-

<150>  JP2004-230139
<151>  2004-08-06

<160>  6

<170>  PatentIn version 3.1

<210>  1
<211>  1374
<212>  DNA
<213>  Streptococcus mutans

<220>
<221>  CDS
<222>  (1)..(1371)
<223>

<400>  1
atg agt aaa atc gtt att gtt gga gct aac cat gca ggt aca gct gcc      48
Met Ser Lys Ile Val Ile Val Gly Ala Asn His Ala Gly Thr Ala Ala
1               5                   10                  15

att aat act att cta gat aat tac ggt agt gaa aac gaa gtt gtc gtt      96
Ile Asn Thr Ile Leu Asp Asn Tyr Gly Ser Glu Asn Glu Val Val Val
            20                  25                  30

ttt gac caa aat tct aat att tca ttc ttg ggt tgt gga atg gca ctt      144
Phe Asp Gln Asn Ser Asn Ile Ser Phe Leu Gly Cys Gly Met Ala Leu
        35                  40                  45

tgg att gga aaa caa ata tca ggc cct caa ggt ctt ttt tat gct gac      192
Trp Ile Gly Lys Gln Ile Ser Gly Pro Gln Gly Leu Phe Tyr Ala Asp
    50                  55                  60

aag gaa tcg tta gaa gca aaa ggt gct aaa att tat atg gaa tcg cca      240
Lys Glu Ser Leu Glu Ala Lys Gly Ala Lys Ile Tyr Met Glu Ser Pro
65                  70                  75                  80

gtg aca gcc att gat tat gat gct aag agg gtt act gct ttg gtc aat      288
Val Thr Ala Ile Asp Tyr Asp Ala Lys Arg Val Thr Ala Leu Val Asn
                85                  90                  95

ggt caa gaa cat gtt gaa agc tat gag aag ctt att ttg gca aca gga      336
Gly Gln Glu His Val Glu Ser Tyr Glu Lys Leu Ile Leu Ala Thr Gly
            100                 105                 110

tca aca cca atc tta cca cct atc aaa ggt gca gct atc aaa gaa ggt      384
Ser Thr Pro Ile Leu Pro Pro Ile Lys Gly Ala Ala Ile Lys Glu Gly
        115                 120                 125

agt cgt gat ttt gaa gca act ttg aaa aat ctt caa ttt gtt aaa ttg      432
Ser Arg Asp Phe Glu Ala Thr Leu Lys Asn Leu Gln Phe Val Lys Leu
    130                 135                 140

tat caa aat gca gaa gat gtt att aat aaa tta cag gat aag agt caa      480
Tyr Gln Asn Ala Glu Asp Val Ile Asn Lys Leu Gln Asp Lys Ser Gln
145                 150                 155                 160

aat ctg aat cgt att gct gtt gtt ggt gct ggt tat att ggt gta gaa      528
Asn Leu Asn Arg Ile Ala Val Val Gly Ala Gly Tyr Ile Gly Val Glu
                165                 170                 175

ctt gct gaa gcc ttt aaa cgc ctc gga aaa gaa gtg att ctt att gat      576
Leu Ala Glu Ala Phe Lys Arg Leu Gly Lys Glu Val Ile Leu Ile Asp
            180                 185                 190

gtt gtt gat act tgc tta gct ggt tat tat gat cag gat ctt tca gaa      624
Val Val Asp Thr Cys Leu Ala Gly Tyr Tyr Asp Gln Asp Leu Ser Glu

195                 200                 205

atg atg cgt caa aat ttg gaa gat cat ggt att gaa tta gca ttc gga        672
Met Met Arg Gln Asn Leu Glu Asp His Gly Ile Glu Leu Ala Phe Gly
    210                 215                 220

gaa act gtc aaa gcc att gaa ggt gat ggt aaa gtc gaa cgt att gta        720
Glu Thr Val Lys Ala Ile Glu Gly Asp Gly Lys Val Glu Arg Ile Val
225                 230                 235                 240

act gat aaa gcg agc cat gat gtg gat atg gtt att tta gct gtc ggt        768
Thr Asp Lys Ala Ser His Asp Val Asp Met Val Ile Leu Ala Val Gly
                245                 250                 255

ttc cgt cct aat act gca ctt ggc aac gct aaa ctc aaa acc ttc cgt        816
Phe Arg Pro Asn Thr Ala Leu Gly Asn Ala Lys Leu Lys Thr Phe Arg
            260                 265                 270

aat ggt gct ttc ctt gtt gat aaa aaa caa gag aca agt att cct gac        864
Asn Gly Ala Phe Leu Val Asp Lys Lys Gln Glu Thr Ser Ile Pro Asp
        275                 280                 285

gtt tat gcc atc ggc gat tgc gcg act gtt tat gac aac gct att aat        912
Val Tyr Ala Ile Gly Asp Cys Ala Thr Val Tyr Asp Asn Ala Ile Asn
    290                 295                 300

gat acc aat tat att gcc tta gct tca aac gct ctt cgc tca ggt att        960
Asp Thr Asn Tyr Ile Ala Leu Ala Ser Asn Ala Leu Arg Ser Gly Ile
305                 310                 315                 320

gta gct ggt cat aat gca gca ggg cat aaa ttg gaa tct ctt ggt gtt        1008
Val Ala Gly His Asn Ala Ala Gly His Lys Leu Glu Ser Leu Gly Val
                325                 330                 335

caa ggt tca aat ggt att tca att ttt ggt ctc aat atg gtt tca act        1056
Gln Gly Ser Asn Gly Ile Ser Ile Phe Gly Leu Asn Met Val Ser Thr
            340                 345                 350

ggg tta aca caa gaa aaa gca aag cgt ttt ggc tat aat cca gaa gtc        1104
Gly Leu Thr Gln Glu Lys Ala Lys Arg Phe Gly Tyr Asn Pro Glu Val
        355                 360                 365

act gca ttt aca gat ttt cag aag gct agt ttt att gaa cat gat aat        1152
Thr Ala Phe Thr Asp Phe Gln Lys Ala Ser Phe Ile Glu His Asp Asn
    370                 375                 380

tat cct gtt aca ctt aaa att gtc tat gat aag gat agc cga ctg gtt        1200
Tyr Pro Val Thr Leu Lys Ile Val Tyr Asp Lys Asp Ser Arg Leu Val
385                 390                 395                 400

ctt ggt gca caa atg gca tct aaa gaa gat atg tca atg gga att cat        1248
Leu Gly Ala Gln Met Ala Ser Lys Glu Asp Met Ser Met Gly Ile His
                405                 410                 415

atg ttt tca ttg gct att cag gaa aaa gtt acc att gaa cgt tta gct        1296
Met Phe Ser Leu Ala Ile Gln Glu Lys Val Thr Ile Glu Arg Leu Ala
            420                 425                 430

cta ctg gac tat ttc ttt ctt cct cat ttc aat caa ccc tat aat tat        1344
Leu Leu Asp Tyr Phe Phe Leu Pro His Phe Asn Gln Pro Tyr Asn Tyr
        435                 440                 445

atg acc aaa gca gca tta aaa gct aaa tga                                1374
Met Thr Lys Ala Ala Leu Lys Ala Lys
    450                 455

<210>  2
<211>  457
<212>  PRT
<213>  Streptococcus mutans

<400>  2

Met Ser Lys Ile Val Ile Val Gly Ala Asn His Ala Gly Thr Ala Ala
1               5                   10                  15

Ile Asn Thr Ile Leu Asp Asn Tyr Gly Ser Glu Asn Glu Val Val Val
            20                  25                  30

Phe Asp Gln Asn Ser Asn Ile Ser Phe Leu Gly Cys Gly Met Ala Leu
        35                  40                  45

Trp Ile Gly Lys Gln Ile Ser Gly Pro Gln Gly Leu Phe Tyr Ala Asp
    50                  55                  60

Lys Glu Ser Leu Glu Ala Lys Gly Ala Lys Ile Tyr Met Glu Ser Pro
65                  70                  75                  80

Val Thr Ala Ile Asp Tyr Asp Ala Lys Arg Val Thr Ala Leu Val Asn
                85                  90                  95

Gly Gln Glu His Val Glu Ser Tyr Glu Lys Leu Ile Leu Ala Thr Gly
            100                 105                 110

Ser Thr Pro Ile Leu Pro Pro Ile Lys Gly Ala Ala Ile Lys Glu Gly
        115                 120                 125

Ser Arg Asp Phe Glu Ala Thr Leu Lys Asn Leu Gln Phe Val Lys Leu
    130                 135                 140

Tyr Gln Asn Ala Glu Asp Val Ile Asn Lys Leu Gln Asp Lys Ser Gln
145                 150                 155                 160

Asn Leu Asn Arg Ile Ala Val Val Gly Ala Gly Tyr Ile Gly Val Glu
                165                 170                 175

Leu Ala Glu Ala Phe Lys Arg Leu Gly Lys Glu Val Ile Leu Ile Asp
            180                 185                 190

Val Val Asp Thr Cys Leu Ala Gly Tyr Tyr Asp Gln Asp Leu Ser Glu
        195                 200                 205

Met Met Arg Gln Asn Leu Glu Asp His Gly Ile Glu Leu Ala Phe Gly
    210                 215                 220

Glu Thr Val Lys Ala Ile Glu Gly Asp Gly Lys Val Glu Arg Ile Val
225                 230                 235                 240

Thr Asp Lys Ala Ser His Asp Val Asp Met Val Ile Leu Ala Val Gly
                245                 250                 255

Phe Arg Pro Asn Thr Ala Leu Gly Asn Ala Lys Leu Lys Thr Phe Arg
            260                 265                 270

Asn Gly Ala Phe Leu Val Asp Lys Lys Gln Glu Thr Ser Ile Pro Asp
        275                 280                 285

Val Tyr Ala Ile Gly Asp Cys Ala Thr Val Tyr Asp Asn Ala Ile Asn
    290                 295                 300

Asp Thr Asn Tyr Ile Ala Leu Ala Ser Asn Ala Leu Arg Ser Gly Ile
305                 310                 315                 320

Val Ala Gly His Asn Ala Ala Gly His Lys Leu Glu Ser Leu Gly Val
                325                 330                 335

Gln Gly Ser Asn Gly Ile Ser Ile Phe Gly Leu Asn Met Val Ser Thr
            340                 345                 350

Gly Leu Thr Gln Glu Lys Ala Lys Arg Phe Gly Tyr Asn Pro Glu Val
        355                 360                 365

Thr Ala Phe Thr Asp Phe Gln Lys Ala Ser Phe Ile Glu His Asp Asn
    370                 375                 380

Tyr Pro Val Thr Leu Lys Ile Val Tyr Asp Lys Asp Ser Arg Leu Val
385                 390                 395                 400

Leu Gly Ala Gln Met Ala Ser Lys Glu Asp Met Ser Met Gly Ile His
                405                 410                 415

Met Phe Ser Leu Ala Ile Gln Glu Lys Val Thr Ile Glu Arg Leu Ala
            420                 425                 430

Leu Leu Asp Tyr Phe Phe Leu Pro His Phe Asn Gln Pro Tyr Asn Tyr
        435                 440                 445

Met Thr Lys Ala Ala Leu Lys Ala Lys
    450                 455

<210> 3
<211> 31
<212> DNA
<213> Artificial

<220>
<223> primer-1

<400> 3
gaggatttgc atatgagtaa aatcgttatt g 31

<210> 4
<211> 31
<212> DNA
<213> Artificial

<220>
<223> primer-2

<400> 4
atgaaaacat gtgaattccc attgacatat c 31

<210> 5
<211> 31
<212> DNA
<213> Artificial

<220>
<223> primer-3

<400> 5
gatatgtcaa tgggaattca catgttttca t 31

<210> 6
<211> 31

<212>  DNA
<213>  Artificial

<220>
<223>  primer-4

<400>  6
tttctgcagt tatcatttag cttttaatgc t                                    31

## Description

### Technical Field

The present invention relates to, in an oxidation reaction by dehydrogenase using nicotinamide adenine dinucleotide (hereinafter to be abbreviated as NAD+) as a coenzyme, a method of regenerating coenzyme NAD+ using a Streptococcus-derived water-forming NADH oxidase, and a method of producing an optically active compound from an enantiomer mixture utilizing the regeneration method of NAD+.

### Cross Reference to Related Application

The whole disclosure of Japanese patent application No. 2004-230139 filed on August 6, 2004 including the specification, claims, drawings and abstract are incorporated hereinto by reference.

### Background Art

The oxidoreductases (e.g., dehydrogenase) classified into E.C.1.1 and E.C.1.4 include those that catalyze a reaction to oxidize alcohol and amino acid to ketone and keto acid in the presence of a coenzyme such as NAD+ and the like. In most cases, since the oxidation reaction by such dehydrogenase is stereoselective, when an enantiomer mixture of alcohols or amino acids is reacted with such enzyme, only one enantiomer is oxidized and the remaining enantiomer is enriched. This reaction is an effective method for the production of an optically active compound.

In the above-mentioned oxidation reaction, however, oxidized coenzyme NAD+ is converted to NADH. Thus, the reaction requires a stoichiometric amount of NAD+. As shown in the following reaction scheme 1, however, it is possible to avoid the use of a stoichiometric amount of NAD+ and reduce the amount thereof to be used, by the coupling of a reaction to convert NADH produced by an oxidation reaction by dehydrogenase to NAD+ (NAD+ regeneration system) (in reaction scheme 1, * shows an asymmetric carbon, and its absolute configuration may be (R) or (S)).

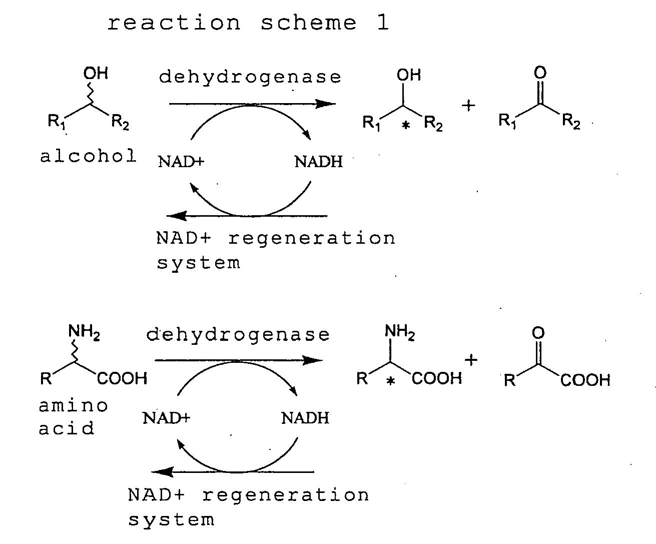

Synthesis methods of optically active compounds utilizing a *Lactobacillus brevis*-derived water-forming NADH oxidase in a coenzyme regeneration system have been reported heretofore (patent reference 1, non-patent references 1, 2). Oxidase is an enzyme that catalyzes the reaction to oxidize NADH with oxygen to produce NAD+ and water, and the reaction essentially requires the presence of oxygen. The reaction to produce NAD+ from NADH by a water-forming NADH oxidase is irreversible. In addition, as the reaction product other than NAD+, only water is produced and the use of the enzyme for the NAD+ regeneration system is preferable.

However, since a *Lactobacillus brevis*-derived water-forming NADH oxidase shows high sensitivity to oxidation, the stabilization thereof requires addition of DTT (dithiothreitol) and the like (non-patent reference 1). Accordingly, addition of a stabilizer such as DTT and the like to the reaction system is necessary even when the water-forming NADH oxidase derived from a microorganism is used for the NAD+ regeneration system.

While a synthesis method of an optically active compound utilizing, other than a *Lactobacillus brevis, Lactobacillus sanfranciscensis-*derived water-forming NADH oxidase as the NAD+ regeneration enzyme has been reported, DTT is also added to the reaction system (non-patent reference 3).
patent reference 1: JP-A-2003-116585
non-patent reference 1: Enzyme and Microbial Technology 32, 205-211 (2003)
non-patent reference 2: Org. Lett. Vol. 5, No 20, 3649-3650 (2003)
non-patent reference 3: Adv. Synth. Catal. 345, 707-712 (2003)

### Disclosure of the Invention

### Problems to be Solved by the Invention

When conventional *Lactobacillus brevis* and *Lactobacillus sanfranciscensis*-derived water-forming NADH oxidases are used as NAD+ regeneration enzymes are used, addition of DTT to the reaction system is generally necessary. When addition of DDT is necessary, problems occur such as (1) extra cost for DDT addition, (2) possibility of reaction with added DTT to reduce the yield, depending on the substrate to be used for the reaction and the resultant product, (3) decreased DTT concentration and decreased protection effect for NADH oxidase as a result of the reaction with DDT and the like. These problems need to be particularly considered, for example, for application thereof to industrial production methods.

The present invention aims at provision of an efficient production method of a useful substance such as optically active alcohol, amino acid and the like, which comprises use of a water-forming NADH oxidase, which is superior in the activity, stability and the like and does not require an enzyme protector such as DTT and the like particularly during the reaction, in the NAD+ regeneration system for the stereoselective oxidation reaction by dehydrogenase.

### Means of Solving the Problems

The present inventors have found, with regard to the above-mentioned problems, a microorganism belonging to the genus *Streptococcus*, more particularly, *Streptococcus mutans*-derived NADH oxidase, does not require addition of a protector such as DTT and the like for stabilization and is highly advantageous as an NAD+ regeneration enzyme, which resulted in the completion of the present invention.
(1) The present invention provides a production method of an enantiomer-enriched compound, which comprises the following (1) and (2):
(1) contacting an enantiomer mixture with a stereoselective dehydrogenase, for which NAD+ (nicotinamide adenine dinucleotide) is a coenzyme, thereby preferentially oxidizing one enantiomer and leaving the other enantiomer to form NADH, and
(2) contacting the NADH formed by the oxidation reaction in (1) with a water-forming NADH oxidase stable under oxidative conditions to convert the NADH to NAD+.

The present invention further provides the following.
(10) A method for utilizing a water-forming NADH oxidase, which is stable without an enzyme protector even in the presence of oxygen, to re-convert NADH formed by the aforementioned reaction to NAD+ in a reaction for preferentially oxidizing one stereo-enantiomer in an enantiomer mixture by a stereoselective dehydrogenase, for which NAD+ (nicotinamide adenine dinucleotide) is a coenzyme.
(11) A water-forming NADH oxidase stable under oxidative conditions, which can be used in combination with stereoselective dehydrogenase, for which NAD+ is a coenzyme.
(12) A water-forming NADH oxidase for a coenzyme regeneration system, which has a coenzyme-regenerative function to reconvert NADH formed by a dehydrogenase reaction with NAD+ as a coenzyme to NAD+, and can be used without an enzyme protector.
(13) A vector comprising a gene of a stereoselective dehydrogenase, for which NAD+ is a coenzyme, and a gene of a water-forming NADH oxidase stable under oxidative conditions.
(14) A transformant microorganism transformed with a vector comprising a gene of a stereoselective dehydrogenase, for which NAD+ is a coenzyme, and a gene of a water-forming NADH oxidase stable under oxidative conditions.
(15) A composition comprising a stereoselective dehydrogenase, for which NAD+ is a coenzyme, and a water-forming NADH oxidase stable under oxidative conditions.

### Effect of the Invention

Using a water-forming NADH oxidase derived from a microorganism belonging to the genus *Streptococcus* for a NAD+ regeneration system, regenerative reaction of NAD+ proceeds efficiently even without adding an enzyme stabilizer such as a reducing agent and the like. Accordingly, when the NAD+ regeneration system is coupled with a stereoselective oxidation reaction by dehydrogenase, an enantiomer-enriched compound can be obtained efficiently from an enantiomer mixture.

### Brief Description of the Drawing

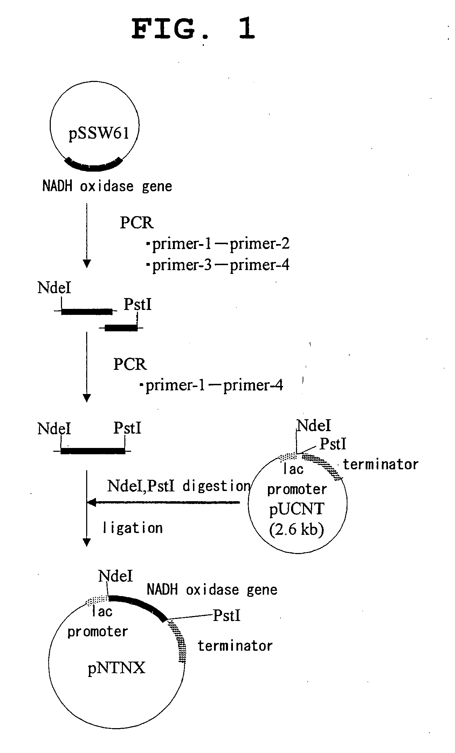

[Fig. 1] A drawing showing a method of constructing a recombinant vector pNTNX as an embodiment.

### Best Mode for Embodying the Invention

### 1. water-forming NADH oxidase

As the "water-forming NADH oxidase" to be used for the NAD regeneration system of the present invention, one that is stable under oxidative conditions, namely, one free of deactivation, or one wherein remarkable deactivation is suppressed, is used.

In the present specification, being "stable under oxidative conditions" is a concept including catalyzing a reaction to convert NADH to NAD+ without remarkable deactivation in the presence of oxygen, for example, under oxygen supply conditions such as in a buffer without particular deaeration, shaking a test tube containing an enzyme solution and the like. In addition, the concept of being "stable under oxidative conditions" includes a condition stable in the presence of oxygen even without utilizing an enzyme protector as if an enzyme protector was used.

The "water-forming NADH oxidase stable under oxidative conditions" to be used in the present invention maintains not less than about 70%, preferably not less than about 80%, more preferably not less than about 90%, of the activity in the absence of an enzyme protector such as DTT and the like, in the presence of oxygen at about 20°C for about 3 hr.

The origin of the "water-forming NADH oxidase" of the present invention is, for example, a microorganism belonging to the genus *Streptococcus,* preferably *Streptococcus mutans,* more preferably *Streptococcus mutans* NCIB11723. The amino acid sequence of Streptococcus *mutans* NCIB11723-derived water-forming NADH oxidase, and the base sequence of DNA encoding the same have already been reported (patent reference: JP-A-8-196281).

SEQ ID NOs: 1, 2 show the amino acid sequence of *Streptococcus mutans* NCIB11723-derived water-forming NADH oxidase, and the base sequence of DNA encoding the same. The *Streptococcus* may be a wild strain or a mutant strain. The mutant strain can be obtained by UV irradiation or a treatment with a mutating agent such as N-methyl-N'nitro-N-nitrosoguanidine (NTG), methylethanesulfonate (EMS) and the like by a conventional method. Any mutant strain can be used as a source of NADH oxidase to be used in the present invention, as long as it can produce the NADH oxidase of the present invention.

Unlike the conventional *Lactobacillus*-derived enzymes, the water-forming NADH oxidase derived from a microorganism belonging to the genus *Streptococcus* does not require addition of a reducing agent such as DTT and the like for stabilization, and is preferably used for the NAD+ regeneration system, which is the object of the present invention. The water-forming NADH oxidase can be obtained by preparing an enzyme from a culture medium of *Streptococcus* bacterium. In addition, it can be obtained easily in a large amount by incorporating a DNA encoding the enzyme into a vector, introducing the vector into a host and expressing the enzyme gene in the obtained transformant.

In the present invention, as the "water-forming NADH oxidase", an enzyme protein substantially the same as the enzyme protein having the amino acid sequence shown in SEQ ID NO: 1 can be used. As for the "substantially the same" enzyme protein, the enzyme protein can be said to be substantially the same when 1, 2, 3, 4, 5, 6, 7, 8, 9, 10 or more substitutions, deletions and/or insertions are introduced into the amino acid sequence of the enzyme protein and the enzyme protein shows an equivalent function. In addition, the enzyme protein to be used in the present invention can be said to be substantially the same when the enzyme protein shows a homology in the amino acid sequence of not less than 60%, preferably not less than 80%, more preferably not less than 90%, and most preferably not less than 99%, with the amino acid sequence shown in SEQ ID NO: 1, and has an equivalent function with the enzyme protein having the amino acid sequence shown in SEQ ID NO: 1.

The above-mentioned "homology" of the amino acid sequence can be determined by comparison with the optimally aligned comparison object sequence. The comparison object sequence may contain addition or deletion after optimal alignment. The homology of amino acid sequences can be calculated, for example, by the Identity value when two amino acid sequences are compared using a sequence similarity searching program FASTA (W.R. Pearson & D.J. Lipman P.N.A.S. (1988) 85:2444-2448) or BLAST (Altschul et. al., J. Mol. Biol. (1990) 215:403-410). The software for the BLAST analysis is publicly available from National Center for Biotechnology Information (http://www.ncbi.nih.gov/).

The enzyme protein having an equivalent function can be encoded by a DNA containing a nucleotide sequence that hybridizes, under stringent conditions, to a nucleotide chain having a sequence complementary to the nucleotide sequence encoding the enzyme protein.

Examples of the hybridization conditions under stringent conditions preferably include: hybridization in about 7% sodium dodecyl sulfate (SDS), about 0.5 M sodium phosphate, 1 mM EDTA at about 50°C, and washing with about 2XSSC, about 0.1% SDS at 50°C; more desirably hybridization in about 7% sodium dodecyl sulfate (SDS), about 0.5 M sodium phosphate, about 1 mM EDTA at 50°C, and washing with about 1XSSC, about 0.1% SDS at about 50°C; more desirably hybridization in about 7% sodium dodecyl sulfate (SDS), about 0.5 M sodium phosphate, about 1 mM EDTA at about 50°C, and washing with about 0.5XSSC, about 0.1% SDS at about 50°C; more preferably hybridization in about 7% sodium dodecyl sulfate (SDS), about 0.5 M sodium phosphate, about 1 mM EDTA at about 50°C, and washing with about 0.1XSSC, about 0.1% SDS at about 50°C; and more preferably about 7% sodium dodecyl sulfate (SDS), about 0.5 M sodium phosphate, about 1 mM EDTA at about 50°C, and washing with about 0.1XSSC, about 0.1% SDS at about 65°C. However, the conditions may vary depending on the length of the nucleotide chain, the sequence, and different environmental parameters. A longer sequence specifically hybridizes at a higher temperature.

A detailed guide of hybridization of nucleic acid can be found, for example, in Tijssen (1993) Laboratory Techniques in Biochemistry and Molecular Biology-Hybridization with Nucleic Acid Probes part I chapter 2, "Overview of principles of hybridization and the strategy of nucleic acid probe assay", Elsvier, New York.

### 2. vector

The vector to be used for obtaining the above-mentioned transformant, any can be used as long as it can express the enzyme gene of the present invention in an appropriate host. As such vector, for example, plasmid vector, phage vector, cosmid vector and the like can be mentioned. Moreover, a shuttle vector capable of gene exchange with other hosts can also be used. Such vector contains regulatory elements such as operably linked promoters (e.g., lacUV5 promoter, trp promoter, trc promoter, tac promoter, lpp promoter, tufB promoter, recA promoter, pL promoter) and the like, and can be preferably used as an expression vector containing an expression unit operably linked to the DNA of the present invention. For example, pUCNT (WO94/03613) and the like can be preferably used.

The term "regulatory element" used in the present specification refers to a functional promoter and a base sequence having any related transcription elements (e.g., enhancer, CCAAT box, TATA box, SPI site and the like). The term "operably linked" used in the present specification means that DNA and various control elements such as a promoter, an enhancer and the like that control the expression thereof are so operably linked in the host cell as to express the gene. It is a well-known matter to those of ordinary skill in the art that the type and kind of the regulatory element may vary depending on the host.

### 3. Transformant

As the host into which a recombinant vector containing the DNA having the gene of the present invention is introduced, bacterium, yeast, fungi, plant cell, animal cell and the like can be mentioned, and *Escherichia coli* is particularly preferable. The DNA of the present invention can be introduced into a host cell by a conventional method. When *Escherichia coli* is used as a host cell, for example, the DNA of the present invention can be introduced by a calcium chloride method.

The transformant (or recombinant) in the present specification means a host organism into which a heterologous nucleic acid molecule is introduced. In general, a nucleic acid molecule is stably incorporated into the host genome or present with an extrachromosomal molecule. Such extrachromosomal molecule can replicate autonomously. A transformed cell, tissue or organism is contained not only in the final product of the transformation process but also in its transgenic progeny.

### 4. reaction

In one embodiment of the present invention, an enantiomer enrichment reaction by stereoselective oxidation by dehydrogenase characteristically utilizes an NAD+ regeneration system using a *Streptococcus* bacterium-derived water-forming NADH oxidase. Therefore, in one aspect, the present invention is characterized by the coupling of a stereoselective oxidation reaction and a NAD+ regeneration system using a Streptococcus-derived water-forming NADH oxidase, where the present invention is not at all limited by the kind of the enantiomer-enriched compound to be produced, and further, the kind of the dehydrogenase to be used for the reaction.

### 5. substrate

The starting material of the enantiomer-enriched compound to be produced by the present invention includes an enantiomer mixture. As the "enantiomer mixture" of the present invention, a compound having an asymmetric carbon wherein the carbon is bonded to hydroxyl group, amino group, formyl group and the like that can be oxidized by dehydrogenase can be mentioned. Specifically, alcohol derivatives inclusive of diol derivative and the like, hydroxy acid derivative, amino acid derivative and the like can be mentioned.

The "derivative" means a compound produced due to a structural change in a small part of a certain compound. A compound wherein hydrogen atom or a particular atomic group in the compound has been substituted by other atom or atomic group is considered to be a derivative of the compound.

### 6. dehydrogenase

For production of the above-mentioned alcohol derivative and hydroxy acid derivative, any enzyme group classified as E.C.1.1.1, which shows enantiomer selective reaction and can utilize NAD+ as a coenzyme, can be used. For production of the above-mentioned amino acid derivative, any enzyme group classified as E.C.1.4.1, which shows enantiomer selective reaction and can utilize NAD+ as a coenzyme, can be used. As an example, dehydrogenase derived from *Celluromonas* sp. strain we have previously found (Japanese Patent Application No. 2004-182926) is an NAD dependent enzyme having a stereoselective oxidation activity to (R)-forms of 1,2-butanediol and 1,3-butanediol. Moreover, the above-mentioned enzyme has a stereoselective oxidation activity to an (S)-form of 3-chloro-1,2-propanediol.

Accordingly, when the above-mentioned enzyme is used as the dehydrogenase of the present invention, an (S)-form enantiomer enriched 1,2-butanediol or 1,3-butanediol can be obtained from an enantiomer mixture of the above-mentioned diol. Moreover, (when the above-mentioned enzyme is used as the dehydrogenase of the present invention,) an (R)-form enantiomer enriched 3-chloro-1,2-propanediol can be obtained from an enantiomer mixture of the above-mentioned diol.

In addition, a dehydrogenase derived from *Candida maris* IFO10003 strain (WO01/005996) catalyzes a stereoselective oxidation reaction of (R) form of 1-phenylethanol.

Therefore, when the above-mentioned enzyme is used as the dehydrogenase of the present invention, an enantiomer-enriched compound in an (S)-form can be obtained from an enantiomer mixture of 1-phenylethanol. Moreover, when an L-form or D-form-specific amino acid dehydrogenase is used, an enantiomer-enriched amino acid derivative in a D-form or an L-form can be obtained from an enantiomer mixture of the amino acid derivative, respectively.

### 7. enantiomer enrichment

The "enantiomer-enriched compound" in the present invention means a compound containing the object optical enantiomer in a proportion of not less than 50% by mol, preferably not less than about 70%, more preferably not less than about 90%, in a mixture with the other optical enantiomer.

The enantiomer enrichment reaction in the present invention can be performed as in the following. First of all, an enantiomer mixture, a dehydrogenase having an oxidation activity on only one of the enantiomers, a water-forming NADH oxidase and NAD+ are added to a suitable solvent and the mixture is stirred.

The water-forming NADH oxidase of the present invention can be used in combination with a stereoselective dehydrogenase, for which NAD+ is a coenzyme. As used herein, "used in combination" means a case where both enzymes of water-forming NADH oxidase and dehydrogenase are simultaneously added to the reaction mixture, and a case where both enzymes are added at different time points as long as they coexist in the reaction mixture.

While the reaction conditions vary depending on the substrate, oxidoreductase and the like to be used, the reaction is generally carried out at a temperature of about 5-80°C, preferably about 10-50°C, at a pH of about 4-10, preferably about 5-9. The enantiomer mixture to be the substrate can be added at a charge concentration of about 0.1-80% (w/v), preferably about 1-60%. NAD+ is added at a concentration of about 0.00001-1 mol%, preferably about 0.00001-0.1% (w/v), relative to the substrate. Since oxygen is necessary for the reaction of NADH oxidase, the reaction mixture is preferably reacted in the air or in the presence of comparatively pure oxygen. To accelerate dissolution of oxygen in the reaction mixture, the reaction is preferably carried out by shaking or under stirring conditions. Furthermore, a reaction under pressurization at not less than the atmospheric pressure may afford more efficient progress of the reaction, since the solubility of oxygen in the reaction mixture is improved.

### 8. enzyme

The enzyme to be used in the present invention, such as dehydrogenase and water-forming NADH oxidase may be a uniformly or partially purified enzyme, and a culture of an organism (e.g., microorganism) that produces such enzyme and a treated product of the culture can also be used. As used herein, the "culture of microorganism" means a culture medium containing a bacterium or cultivated bacterium, and the "treated product" means, for example, a crude extract, lyophilized bacterium, acetone dry bacterium, milled cells thereof, a mixture thereof and the like. Furthermore, they can be used as the enzyme itself or bacterium itself after being immobilized by a known means (e.g., crosslinking method, physical adsorption method, entrapment and the like).

When a reaction is to be carried out, moreover, by the use of a culture of a transformant wherein both the dehydrogenase and the water-forming NADH oxidase have been introduced into the same host cell (e.g., transformant microorganism) or a treated product of the culture, an enantiomer-enriched compound can be produced at a lower cost since microorganisms separately expressing the both enzymes do not need to be cultivated.

In addition, for example, using a transformant microorganism expressing the both enzymes in the same cell, the reaction can proceed using the intracellular NAD+ of the microorganism, which in turn obviates separate addition of NAD+ from the outside, or the amount of NAD+ to be added can be drastically reduced. Such transformant can be produced by incorporating a DNA encoding the dehydrogenase to be used and a DNA encoding the water-forming NADH oxidase into the same vector, and introducing the vector into a host. Such transformant can also be produced by respectively incorporating these two kinds of DNAs into two kinds of vectors of different incompatible groups and introducing the vectors into the same host.

In other words, a transformant containing a recombinant vector containing a DNA encoding a dehydrogenase to be used and a DNA encoding a NADH oxidase, and a transformant containing a first recombinant vector containing a DNA encoding a dehydrogenase to be used and a second recombinant vector containing a DNA encoding a NADH oxidase can be used.

The "composition" of the present invention, which comprises a stereoselective dehydrogenase, for which NAD+ is a coenzyme, and a water-forming NADH oxidase is produced by mixing the both enzymes and, where desired, a carrier. Such composition can also contain other active substances and conventional additives, for example, stabilizer, wetting agent, emulsifier, flavor, buffer and the like. The composition of the present invention can be used for the method of the present invention.

The present invention is explained in more detail in the following by referring to Examples, which are not to be construed as limitative.

### Examples

### (Example 1) Preparation of recombinant vector containing NADH oxidase gene and recombinant Escherichia coli

For expression of *Streptococcus mutans*-derived water-forming NADH oxidase in *Escherichia coli*, a recombinant vector used for transformation was prepared. First, a double stranded DNA, wherein a NdeI site was added to the starting point of a NADH oxidase structural gene, and a new stop codon and a PstI site were added to immediately after the stop codon, was obtained by the following method.

PCR was performed using a combination of synthetic primers depicted by 5'-gaggatttgcatatgagtaaaatcgttattg-3' (primer-1: SEQ ID NO: 3) and 5'-atgaaaacatgtgaattcccattgacatatc-3' (primer-2: SEQ ID NO: 4) and, as a template, plasmid pSSW61 (Biosci. Biotech. Biochem., 60(1), 39-43, 1996) containing water-forming NADH oxidase gene, whereby a double stranded DNA1 was synthesized. Similarly, PCR was performed using a combination of synthetic primers of 5'-gatatgtcaatgggaattcacatgttttcat-3' (primer-3: SEQ ID NO: 5) and 5'-tttctgcagttatcatttagcttttaatgct-3' (primer-4: SEQ ID NO: 6) and, as a template, plasmid pSSW61 (Biosci. Biotech. Biochem., 60(1), 39-43, 1996) containing water-forming NADH oxidase gene, whereby a double stranded DNA2 was synthesized.

Furthermore, PCR was performed using the aforementioned two kinds of synthetic primers (primer-1, primer-4) and, as a template, a mixture of double stranded DNA1 and DNA2 obtained above, whereby a double stranded DNA was obtained. The obtained DNA fragment was digested with NdeI and PstI, and inserted into the NdeI, PstI sites at the downstream of lac promoter of plasmid pUCNT (WO94/03613), whereby a recombinant plasmid pNTNX was obtained.

Fig. 1 shows the preparation method and structure of pNTNX. Using the recombinant vector pNTMX, *Escherichia coli* HB101 (manufactured by Takara Shuzo) was transformed to give recombinant *Escherichia coli* HB101(pNTNX).

### (Example 2) Expression of NADH oxidase by recombinant Escherichia coli

The recombinant *Escherichia coli* HB101(pNTNX) obtained in Example 1 was cultured in 2×YT medium (Bacto Tryptone 1.6% (w/v), Bacto yeast extract 1.0% (w/v), NaCl 0.5% (w/v), 100 µg/ml ampicillin, pH 7.0) supplemented with 100 µg/ml of ampicillin and glycerol 0.8% (w/v), and the cells were harvested, suspended in 100 mM Tris-HCl buffer (pH 7), and disrupted by an ultrasonication disruption method to give a cell-free extract. The specific activity of the NADH oxidase in the cell-free extract was 30 U/mg protein.

### [NADH oxidase activity measurement conditions]

An enzyme solution (0.05 ml) was added to a reaction mixture (0.95 ml) containing 100 mM phosphate buffer (pH 7.0), NADH 0.17 mM, EDTA 0.2 mM and FAD 0.02 mM, and the activity was assayed by the measurement of a decrease in the absorbance at 30°C and wavelength 340 nm. The enzyme activity necessary for oxidation of 1 µmol of NADH to NAD+ in 1 min under such reaction conditions was defined to be 1 unit.

### (Example 3) Preparation of cell-free extract

The recombinant *Escherichia coli* HB101(pNTNX) expressing NADH oxidase obtained in Example 1 was inoculated to 80 ml of the medium described in Example 2, which was sterilized in a 500 ml-Sakaguchi-flask, and subjected to shaking culture at 30°C for 32 hr. After cell harvest, the cells were suspended in 10 ml of 100 mM phosphate buffer (pH 7.0), disrupted by an ultrasonication disruption method, and centrifuged to give a cell-free extract containing NADH oxidase.

Then, *E. coli* HB101(pNTFP) (deposit number FERM BP-7116) expressing Candida maris-derived dehydrogenase and *E. coli* HB101(pTSCS) (FERM BP-10024) expressing *Celluromonas* sp.-derived dehydrogenase were each inoculated to 60 ml of a semi-synthetic medium (glycerol 1.5% (w/v), yeast extract 0.3% (w/v), Na₂HPO₄ 0.6% (w/v), KH₂HPO₄ 0.3% (w/v), NaCl 0.2% (w/v), MGSO4·7H2O 0.5% (w/v), 100 µg/ml ampicillin, pH 6.0), which was sterilized in a 500 ml-Sakaguchi-flask, and subjected to culture at 37°C for 37 hr. After cell harvest, the cells were suspended in 10 ml of 100 mM phosphate buffer (pH 7.0), disrupted by an ultrasonication disruption method, and centrifuged to give a cell-free extract containing two kinds of NAD dependent dehydrogenases. The cell-free extract prepared from HB101(pTSCS) and HB101(pNTFP) showed an oxidation activity of 10 U/mg for 1,2-butanediol and an oxidation activity of 5 U/mg for 1-phenylethanol.

### [1,2-butanediol or 1-phenylethanol oxidation activity measurement conditions]

An enzyme solution (0.05 ml) was added to a reaction mixture (0.95 ml) containing 100 mM phosphate buffer (pH 7.0), 1,2-butanediol or 1-phenylethanol 20 mM, and NAD+ 0.17 mM, and the activity was assayed by the measurement of an increase in the absorbance at 30°C and wavelength 340 nm. The enzyme activity necessary for oxidation of 1 µmol of NADH to NAD+ in 1 min under such reaction conditions was defined to be 1 unit.

*E. coli* HB101(pNTFP) (FERM-7116) was deposited on April 11, 2000, and *E. coli* HB101(pTSCS) (FERM BP-10024) was deposited on May 12, 2004 under each deposit number mentioned above in the International Patent Organism Depositary, National Institute of Advanced Industrial Science and Technology (IPOD: 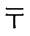 305-8566 Tsukuba Central 6, 1-1-1 Higashi, Tsukuba, Ibaraki Japan).

### (Example 4) Optical resolution of 1,2-butanediol

A reaction mixture (1 ml) containing the HB101(pTSCS) cell-free extract (dehydrogenase) 70 µl and HB101(pNTNX) cell-free extract (NADH oxidase) 28 µl, both prepared in Example 3, 10 mg of racemate of 1,2-butanediol, and 300 mM potassium phosphate buffer (pH 7.0) was shaken in a test tube at 20°C for 23 hr to carry out the reaction. The reaction mixture (200 µl) was saturated with ammonium sulfate, and 1,2-butanediol was extracted with ethyl acetate (400 µl). The solvent was removed from 100 µl of the extract under reduced pressure, trifluoroacetic anhydride (200 µl) was added to the residue and the mixture was stood at room temperature for 10 min to allow trifluoroacetylation, and unreacted trifluoroacetic anhydride was removed under reduced pressure. Ethyl acetate (500 µl) was further added to dissolve trifluorinated 1,2-butanediol, and the optical purity was analyzed by capillary gas chromatography. The above-mentioned enzyme reaction mixture (100 µl) was saturated with ammonium sulfate, 1,2-butanediol was extracted with ethyl acetate (1000 µl), and the 1,2-butanediol content of the extract was analyzed by gas chromatography. As a result, a 1,2-butanediol (S)-form having an optical purity of 97% was produced in a yield of 49%.

### [optical purity analysis conditions]

column: Chiradex G-TA (20 m×0.25 mm) (manufactured by ASTEC)
column temperature: 60°C
injection temperature: 150°C
detection temperature: 150°C
carrier gas: helium (130 kPa)
split ratio: 100/1
detection time: 1,2-butanediol R-form 5.0 min, S-form 5.8 min

### [content analysis conditions]

column: HP-5 30 m×0.32 mm I.D. (manufactured by Agilent Technologies)
detection: FID
   column initial temperature: 50°C, column final temperature: 200°C, temperature rise rate: 6°C/min injection temperature: 150°C
   detection temperature: 300°C
   carrier gas: helium (70 kPa)
   split ratio: 100/1

### (Example 5) Optical resolution of 1-phenylethanol

A reaction mixture (1 ml) containing the HB101(pNTFP) cell-free extract (dehydrogenase) 19 µl and HB101(pNTNX) cell-free extract (NADH oxidase enzyme) 28 µl, both prepared in Example 3, 10 mg of racemate of 1-phenylethanol, and 300 mM potassium phosphate buffer was shaken at 20°C for 23 hr to carry out the reaction. The reaction mixture (100 µl) was saturated with ammonium sulfate, and 1-phenylethanol was extracted with ethyl acetate (700 µl). The optical purity and content of 1-phenylethanol in the extract were analyzed by HPCL (column: manufactured by Daicel Chemical Industries, LTD., Chiralcell OD-H (0.46×25 cm), column temperature: 25°C, eluent: n-hexane/2-propanol=9/1, flow rate: 0.5 ml/min, elution time: (R) form-12.2 min, (S) form-13.3 min). As a result, a 1-phenylethanol (S)-form having an optical purity of 100% was produced in a yield of 48%.

### [optical purity, content analysis conditions]

column: Chiralcell OD-H 0.46×25 cm (manufactured by Daicel Chemical Industries, LTD.)
eluent: n-hexane/2-propanol=9/1
flow rate: 0.5 ml/min
elution time: (R) form-12.2 min, (S) form-13.3 min)

### (Example 6) Optical resolution of 1-phenylethanol (DTT addition, no addition)

A reaction mixture (1 ml) containing the HB101 (pNTFP) cell-free extract (dehydrogenase) 10 µl and HB101(pNTNX) cell-free extract (NADH oxidase) 5 µl, both prepared in Example 3, 10 mg of racemate of 1-phenylethanol, and 300 mM potassium phosphate buffer, and a reaction mixture (1 ml) containing this reaction mixture and 5 mM DTT were shaken at 20°C for 6 hr to carry out the reaction. Thereafter, the content and optical purity of 1-phenylethanol in the reaction mixtures were measured in the same manner as in Example 5. As a result, 1-phenylethanol having an optical purity of 95% was produced in a yield of 50% and 1-phenylethanol having an optical purity of 94% was produced in a yield of 51%, in the reaction mixtures without or with addition of DTT, respectively. As shown above, the same level of reaction proceeded even without addition of DTT to the reaction mixture, as with the addition of DTT, and the addition of DTT was not substantially necessary.

### (Example 7) Stability of water-forming NADH oxidase in the presence of oxygen

An HB101 (pNTNX) cell-free extract (0.1 ml) containing water-forming NADH oxidase, which was prepared in Example 3, was added to 100 mM phosphate buffer (0.9 ml) or 100 mM phosphate buffer (0.9 ml) containing 5 mM DTT, the mixture was stood still or maintained under shaking condition at 300 rpm (oxygen supply condition) in a small test tube at 20°C for 3 hr, and the residual NADH oxidase activity was assayed. The results are shown in Table 1. Under any conditions, the activity of not less than 90% was maintained.

**Table 1**

| conditions | residual activity (%) | |
|---|---|---|
| | standing still | shaking |
| 100 mM phosphate buffer | 92 | 93 |
| 100 mM phosphate buffer (+ 5 mM DTT) | 94 | 93 |

## Claims

1. A production method of an enantiomer-enriched compound, which comprises the following (1) and (2):
(1) contacting an enantiomer mixture with a stereoselective dehydrogenase, for which NAD+ (nicotinamide adenine dinucleotide) is a coenzyme, thereby preferentially oxidizing one enantiomer and leaving the other enantiomer to form NADH, and
(2) contacting the NADH formed by the oxidation reaction in (1) with a water-forming NADH oxidase stable under oxidative conditions to convert the NADH to NAD+.

2. The production method of claim 1, wherein the water-forming NADH oxidase is an enzyme derived from a microorganism belonging to the genus *Streptococcus* or an enzyme substantially the same as said enzyme.

3. The production method of claim 2, wherein the microorganism belonging to the genus *Streptococcus* is *Streptococcus mutans.*

4. The production method of claim 3, wherein the *Streptococcus mutans* is *Streptococcus mutans* NCIB11723 or a mutant strain thereof.

5. The production method of claim 4, wherein the water-forming NADH oxidase is a protein having the amino acid sequence shown in SEQ ID NO: 1 or comprises a protein substantially the same as said protein.

6. The production method of any one of claims 1 to 5, wherein an enzyme source of the water-forming NADH oxidase is a culture of a microorganism capable of recombinant expression of the enzyme or a treated product of the culture.

7. The production method of any one of claims 1 to 5, wherein an enzyme source of the dehydrogenase and water-forming NADH oxidase is a culture of a recombinant microorganism wherein the both enzymes have been expressed by the same host, or a treated product of the culture.

8. The production method of any one of claims 1 to 7, wherein the enantiomer mixture is a secondary alcohol or a derivative thereof.

9. The production method of any one of claims 1 to 7, wherein the enantiomer mixture is an amino acid or a derivative thereof.

10. A method for utilizing a water-forming NADH oxidase, which is stable without an enzyme protector even in the presence of oxygen, to re-convert NADH formed by the aforementioned reaction to NAD+ in a reaction for preferentially oxidizing one stereo-enantiomer in an enantiomer mixture by a stereoselective dehydrogenase, for which NAD+ (nicotinamide adenine dinucleotide) is a coenzyme.

11. A water-forming NADH oxidase stable under oxidative conditions, which can be used in combination with stereoselective dehydrogenase, for which NAD+ is a coenzyme.

12. A water-forming NADH oxidase for a coenzyme regeneration system, which has a coenzyme-regenerative function to reconvert NADH formed by a dehydrogenase reaction with NAD+ as a coenzyme to NAD+, and can be used without an enzyme protector.

13. A vector comprising a gene of a stereoselective dehydrogenase, for which NAD+ is a coenzyme, and a gene of a water-forming NADH oxidase stable under oxidative conditions.

14. A transformant microorganism transformed with a vector comprising a gene of a stereoselective dehydrogenase, for which NAD+ is a coenzyme, and a gene of a water-forming NADH oxidase stable under oxidative conditions.

15. A composition comprising a stereoselective dehydrogenase, for which NAD+ is a coenzyme, and a water-forming NADH oxidase stable under oxidative conditions.
